# EUROPEAN PATENT SPECIFICATION

(11) **EP 1 549 305 B1**
(45) Date of publication and mention of the grant of the patent: **22.04.2009**
(21) Application number: 03774642.7
(22) Date of filing: 07.10.2003
(51) Int. Cl.: A61K 31/17, A61K 31/4174, A61P 25/16, A61P 25/28

(54) **ALPHA 2B 0R 2B/2C ADRENOCEPTOR AGONISTS FOR THE TREATMENT OF MORBUS ALZHEIMER AND MORBUS PARKINSON**
ALPHA 2B ODER 2B/2C ADRENOZEPTOR-AGONISTEN ZUR BEHANDLUNG VON MORBUS ALZHEIMER UND MORBUS PARKINSON
AGONISTES DES RECEPTEURS ADRENERGIQUES ALPHA 2B OU 2B/2C POUR LE TRAITEMENT DE MORBUS ALZHEIMER ET MORBUS PARKINSON

(30) Priority: 08.10.2002 US 417049 P
(43) Date of publication of application: 06.07.2005
(73) Proprietor: ALLERGAN, INC., Irvine CA 92612 (US)
(72) Inventor: WHEELER, Larry, A., Irvine, CA 92612 (US); GIL, Daniel, W., Corona Del Mar, CA 92625 (US); DONELLO, John, E., Dana Point, CA 92629 (US)
(74) Representative: HOFFMANN EITLE
(86) International application number: PCT/US2003/031809
(87) International publication number: WO 2004/032913

(56) References cited:
- WO-A-99/28300
- WO-A-02/076950
- WO-A-03/099795

## Description

The methods of the present invention employ alpha 2 adrenergic receptor-selective agonists to prevent nerve cell damage and death, such as that observed in Parkinson's disease and Alzheimer's disease.

### BACKGROUND OF THE INVENTION

Human adrenergic receptors are integral membrane proteins which have been classified into two broad classes, the alpha and the beta adrenergic receptors. Both types mediate the action of the peripheral sympathetic nervous system upon binding of catecholamines, norepinephrine and epinephrine.

Norepinephrine is produced by adrenergic nerve endings, while epinephrine is produced by the adrenal medulla. The binding affinity of adrenergic receptors for these compounds forms one basis of the classification: alpha receptors tend to bind norepinephrine more strongly than epinephrine and much more strongly than they bind the synthetic compound isoproterenol. The preferred binding affinity of these hormones is reversed for the beta receptors. In many tissues, the functional responses, such as smooth muscle contraction, induced by alpha receptor activation are opposed to responses induced by beta receptor binding.

Subsequently, the functional distinction between alpha and beta receptors was further highlighted and refined by the pharmacological characterization of these receptors from various animal and tissue sources. As a result, alpha and beta adrenergic receptors were further subdivided into alpha 1, alpha 2, beta 1, and beta 2 subtypes.

Furthermore, it has since been recognized that each of these receptors has a number of subtypes; thus the human alpha 2 receptor can be further broken down into the alpha 2A, alpha 2B and alpha 2C receptor subtypes.

Functional differences between alpha 1 and alpha 2 receptors have been recognized, and compounds which exhibit selective binding between these two subtypes have been described.

Thus, in WO 92/00073, the ability of the R(+) enantiomer of terazosin to selectively bind to adrenergic receptors of the alpha 1 subtype was reported. The alpha 1/alpha 2 selectivity of this compound was disclosed as being significant because agonist stimulation of the alpha 2 receptors was said to inhibit secretion of epinephrine and norepinephrine, while antagonism of the alpha 2 receptor was said to increase secretion of these hormones. Thus, the use of non-selective alpha-adrenergic blockers, such as phenoxybenzamine and phentolamine, was said to be limited by their α₂ adrenergic receptor mediated induction of increased plasma catecholamine concentration and the attendant physiological sequelae (increased heart rate and smooth muscle contraction). It is significant that the selectivity of compounds termed "alpha 1 selective" or "alpha 2 selective" have traditionally been based on K_{D} data, which is limited to comparison of binding affinities to receptors, and does not compare the actual biological activities at the compared receptors.

In contrast, a method for measuring alpha receptor agonist selectivity comprises the RSAT (Receptor Selection and Amplification Technology) assay as reported in Messier et al., High Throughput Assays Of Cloned Adrenergic, Muscarinic, Neurokinin And Neurotrophin Receptors In Living Mammalian Cells, Pharmacol. Toxicol. 76:308-11 (1995) and has been adapted for use with alpha 2 receptors. The assay measures a receptor-mediated loss of contact inhibition that results in selective proliferation of receptor-containing cells in a mixed population of confluent cells. The increase in cell number is assessed with an appropriate transfected marker gene such as b-galactosidase, the activity of which can be easily measured in a 96-well format. Receptors that activate the G protein, Gq, elicit this response. Alpha₂ receptors, which normally couple to Gᵢ, activate the RSAT response when coexpressed with a hybrid Gq protein that has a Gi receptor recognition domain, called G_{q}/i5². See Conklin et al., Substitution Of Three Amino Acids Switches Receptor Specificity Of Gqa To That Of Gia, Nature 363:274-6. (1993).

Various alpha adrenergic receptor agonists have been reported as being useful for treating a wide variety of conditions and disorders. Thus, alpha adrenergic receptor agonists such as clonidine have been described and used as systemic and ocular hypotensive agents, as agents useful in the treatment of withdrawal from addictive behaviors such as smoking and drug abuse, and as antidysmenorrheal agents. Another alpha adrenergic receptor agonist, tizanidine, has been used for the treatment of symptoms of spasticity in multiple scelerosis patients by decreasing muscle tone. These agents have also been reported to certain analgesic activities.

These agents, while useful, have been plagued with sometimes serious side effects including sedation, cardiovascular effects such as hypotension and decreased heart rate, and dizziness that have limited their applicability for certain indications. In particular, these agents tend to have overlapping therapeutic and sedation dose response curves, such that sedative activity begins to be noticeable at the same doses as the appearance of therapeutic (e.g., hypotensive or analgesic) activity in vivo.

Compounds such as clonidine tizanidine, and dexmedetomidine have been characterized in the literature as "alpha 2 adrenergic receptor agonists", based largely on binding studies. See also Hieble et al., J. Med Chem. 38:3415 (September 1, 1995); Ruffolo, et al., J. Med. Chem. 38: 3681 (September 15, 1995). While it is true that these agents are alpha 2 receptor agonists, it is not generally appreciated that these agents also contain significant amounts of alpha 1 receptor agonist activity. Nor has the effect of such alpha 1 receptor activity on alpha 2 activity been generally known or appreciated.

By contrast, the compound brimonidine and its functionally similar 2-inidazolin-2-ylimino derivatives (as described below) are alpha 2 agonists which exhibit a markedly greater agonist activity towards the alpha 2 receptors than towards the alpha 1 receptor subtypes.

Additionally, these compounds are now known to be alpha 2 "pan agonists", meaning that little or no functional selectivity is seen in the stimulation of the alpha 2A, alpha 2B and alpha 2C receptor subtypes.

Recently compounds selective or specific for the alpha 2B and/or alpha 2C receptor subtypes, and certain advantages of such compounds, have been disclosed. Thus, for example, United States Patents No.6,329,369 and 6,313,172 and pending, co-owned U.S. patent applications 09/778975, 09/794874 and 10/153328 discloses such compounds, and their use for conditions including pain, muscle spasticity; pain; neurodegenerative diseases, spinal ischemia and stroke; memory and cognition deficits; psychoses, anxiety and depression; hypertension; congestive heart failure; cardiac ischemia and nasal congestion. In these patents, compounds are deemed selective alpha 2B or alpha2B/2C agonists if that compound's difference in efficacy as an agonist of the alpha 2B or alpha 2B/2C receptor subtype(s) compared to the alpha 2A receptor subtype is greater than 0.3 and its efficacy at the alpha 2A receptor subtype is at least approximately 10 times less potent than at the alpha 2B and/or alpha 2C receptor subtype.

CNS disorders are a type of neurological disorder. Several CNS disorders can be attributed to a cholinergic deficiency, a dopaminergic deficiency, an adrenergic deficiency and/or a serotonergic deficiency. CNS disorders of relatively common occurrence include presenile dementia (early onset Alzheimer's disease), senile dementia (dementia of the Alzheimer's type), and Parkinsonism including Parkinson's disease.

The foundation of today's understanding of Alzheimer's disease is based upon the observation that certain regions of the brain of affected persons, such as the hippocampus and the cerebral cortex, showed evidence of a loss of nerve cells. Since the 1970s researchers have known that some of these dying neurons are cholinergic--that is, they communicate using the neurotransmitter acetylcholine, which is ultimately broken down by an enzyme called acetylcholinesterase. See, Jones, et al., Intern. J. Neurosci. 50:147 (1990); Perry, Br. Med. Bull. 42:63 (1986); and Sitaram, et al., Science 201:274 (1978).

Drugs that became available in the past decade, such as tacrine and donepezil, are acetylcholinesterase inhibitors. By preventing the breakdown of acetylcholine, these compounds slow the development of the early stages Alzheimer's disease. However, once cholinergic neurons degenerate fully and can no longer produce acetylcholine neurotransmitter, these drugs become useless.

Besides noting the loss of nerve cells, the brains of patients suffering from Alzheimer's disease characteristically contain clusters of proteins. These accumulations occur in two forms: those found inside neurons and those found in the intercellular space. Intracellular clusters are called neurofibrillary tangles, and appear like pairs of fibers wound around each other in a helix. Analyses have shown that tangles consist of tau protein. Tau is significant because it binds to tubulin, which is responsible for microtubule formation. The number of neurofibrillary tangles appears to correlate with the severity of the disease.

The intercellular protein clusters or plaques are composed of deposits of β-amyloid protein. The nearby neurons often appear swollen and deformed, and the amyloid plaques are usually accompanied by inflammatory microglia. The microglia, which are part of the brain's immune system, may be present in an attempt to degrade and remove damaged neurons or perhaps the plaques themselves.

It is unclear whether the neurons in or near these plaques function normally, because the density of plaques is only weakly correlated with the severity of dementia. Further, such plaques are present in most elderly people, whether they have Alzheimer's disease or not. Nevertheless, their extensive presence in the hippocampus and the cerebral cortex is specific to Alzheimer's patients, and they appear long before neurofibrillary tangles do.

β-amyloid plaques contain a 42 amino acid fragment of an integral membrane protein termed β-amyloid precursor protein (BAPP). This fragment is generated by a two-step cleavage of the BAPP protein, first by a protease termed β secretase and then by gamma secretase. The normal cleavage product of β secretase and gamma secretase is a 40 amino acid peptide, which, unlike the 42 amino acid derivative, does not appear to be involved in the initiation or progression of Alzheimer's disease.

Parkinson's disease (PD) is a debilitating neurodegenerative disease, presently of unknown etiology, characterized by tremors and muscular rigidity. A feature of the disease appears to involve the degeneration of dopaminergic neurons (i.e., which secrete dopamine), particularly in the substantia nigra and ventral tegmental regions of the midbrain. See, Rinne, et al., Brain Res. 54:167 (1991) and Clark, et al., Br. J. Pharm. 85:827 (1985). The substantia nigra is involved in the coordination of neural signals for movements and posture. The ventral tegmental area (VTA) of the midbrain contains neurons which project to sites including the prefrontal cortex, the area of the brain associated with the higher cognitative functions.

Certain attempts have been made to treat PD. One proposed treatment for PD is SINEMET®, which is a sustained-release tablet containing a mixture of carbidopa and levodopa, available from The DuPont Merck Pharmaceutical Co. Another proposed treatment for PD is ELDEPRYL®, which is a tablet containing selefiline hydrochloride, available from Somerset Pharmaceuticals, Inc. Another proposed treatment for PD is PARLODEL®, which is a tablet containing bromocriptine mesylate, available from Sandoz Pharmaceuticals Corporation. Another method for treating PD and a variety of other neurodegenerative diseases through melanin therapy has been proposed in U.S. Pat. No. 5,210,076 to Berliner et al. However, none of these treatments appear to protect neurons from cell death.

### SUMMARY OF THE INVENTION

While it has been known that certain compounds, including certain alpha adrenergic agonists such as brimonidine, are able to provide neuroprotective activity to optic or retinal cells and the nerve cells of the spine when applied topically or injected at the site of nerve damage, it has heretofore not been thought that such agents would be effective agents for the treatment of neurodegenerative conditions of the brain, such as Alzheimer's disease and Parkinson's disease, since it has previously not been thought that alpha 2B and/or alpha 2C receptors were plentiful in these areas of the brain. Additionally, while alpha adrenergic receptor agonists have been shown to have useful neuroprotective activities when administered for topical treatment, the sedative effects of these compounds observed at therapeutic doses have severely limited their usefulness as a practical matter as non-topical or systemic agents.

The present Applicants have surprisingly discovered that certain alpha adrenergic agents are able, when administered systemically, to protect nerve cells of the substantia nigra and the ventral tagmental area of the brain. Moreover, these agents have a dramatically broader therapeutic window between their neuroprotective activity and their sedative activity than most previously characterized alpha adrenergic agonists.

Among this class of drugs are various quinoxaline derivatives having alpha 2 agonist activity which were originally suggested as therapeutic agents by Danielewicz, et al. in U.S. Pat. Nos. 3,890,319 and 4,029,792. These patents disclose compounds as regulators of the cardiovascular system which have the following formula: 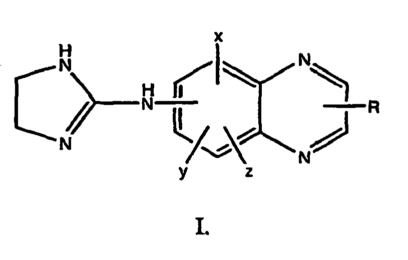 where the 2-imidazolin-2-ylamino group may be in any of the 5-, 6-, 7- or 8-position of the quinoxaline nucleus; x, y and z may be in any of the remaining 5-, 6-, 7- or 8-positions and may be selected from hydrogen, halogen, C₁₋₅ alkyl, C₁₋₅ alkoxy or trifluoromethyl; and R is an optional substituent in either the 2- or 3-position of the quinoxaline nucleus and may be hydrogen, C₁₋₅ alkyl or C₁₋₅ alkoxy. The presently useful compounds may be prepared in accordance with the procedures described in Pat. Nos. 3,890,319 and 4,029,792.

In *Ocular Effects of a Relatively Selective Alpha-2 Agonist (*UK-14,304-18*) in* Cats, Rabbits and Monkeys, J. A. Burke, et al., Current Eye Rsrch., 5, (9), pp. 665-676 (1986) the quinoxaline derivative shown below and having the generic name brimonidine was shown to be effective in reducing intraocular pressure in rabbits, cats and monkeys. Compounds in this study were administered topically to the corneas of the study animals.

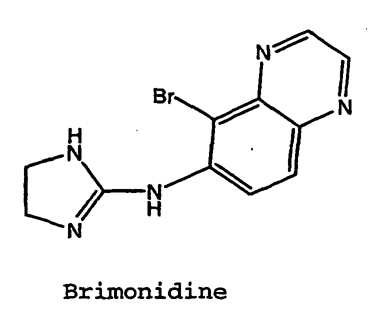

It is known that the alpha 2 receptor agonist brimonidine can protect retinal neural cells, including photoreceptors and retinal ganglion cells, from damage in conditions such as glaucoma, retinitis pigmentosa, and age-related macular degeneration when administered topically or systemically.

In a first aspect the present invention is directed to the use in methods for treating Alzheimer's disease as Parkinson's disease of the brain comprising administering to the brain of a mammal in need thereof a therapeutically effective amount of a selective alpha 2 adrenergic receptor agonist.

As used herein, a "selective alpha 2 adrenergic receptor agonist" or a "selective alpha 2 agonist" shall mean an agent having a ratio of efficacy at an alpha 2 receptor to the efficacy at an alpha 1 receptor greater than that provided by the agent dexmedatomidine. Preferably the efficacy is at least 12 time greater that an alpha 2 receptor than at an alpha 1 receptor; even more preferably the efficacy at the alpha 2 receptor(s) is at least 25 times greater than at an alpha 1 receptor.

In one embodiment, the selective alpha 2 agonist is a 2-imidazolin-2-ylamino compound having the structure shown in Structure I, above. In a preferred embodiment, the selective alpha 2 agonist is brimonidine or its salts.

In another embodiment the selective alpha 2 agonist is also a selective alpha 2B or selective alpha 2B/2C agonist. As used herein, a "selective alpha 2B or 2B/2C agonist", or "selective alpha 2B or 2B/2C adrenergic receptor agonist" means a compound having at least 10-fold (preferably at least 50-fold, even more preferably at least 100-fold) greater efficacy at the alpha 2B receptor, or at both the alpha 2B and alpha 2C receptor subtypes than at the alpha 2A receptor subtype.

Preferably a "selective" compound is "specific", meaning that the compound has at least 100-fold (preferably at least 500-fold; even more preferably at least 1000-fold; yet more preferably at least 5000-fold) greater efficacy at the indicated receptor(s) or receptor subtype(s) than they have at the receptor(s) or receptor subtype(s) they are being compared with.

Efficacy of a given receptor or receptor subtype in accordance with the present invention is determined using the RSAT assay procedure described above.

Selective alpha 2B and 2B/2C agonists are of particular use in the methods of the present invention. Selective alpha 2 agonists exhibit an improved therapeutic index due to diminution of the EC₅₀ of such compounds (leading to a therapeutic effect at a lower concentration of drug) as compared to similar compounds having alpha 1 receptor activity, with no change in the sedation dose-response curve. Selective alpha 2B or 2B/2C agonists additionally have diminished sedative activity by virtue of the diminished alpha 2A receptor activity, which the inventors have discovered is responsible for sedation and cardiovascular effects such as lowered heart rate and blood pressure. These effects are particularly maximized when the compounds are specific rather than merely selective for their specified target.

In another aspect, the present invention is directed to methods for preventing death or degeneration of neural cells projecting to or from a region of the brain selected from the group consisting of the substantia nigra, the locus ceruleus and the ventral tegmental area in Parkinson's as Alzheimer's disease comprising administering a selective alpha 2 adrenergic receptor agonist to said cells. In one embodiment, the selective alpha agonist is also a selective alpha 2B or selective alpha 2B/2C agonist. Preferably, the agents are specific for their specified target.

In yet another aspect, the invention is directed to the use in methods for treating Alzleimer's as Parkinson's disease comprising administering to the brain of a mammal in need thereof a therapeutically effective amount of an alpha 2 adrenergic receptor agonist and an alpha 1 receptor antagonist. Use of an alpha receptor antagonist in conjunction with an alpha 2 receptor agonist will result in a combination medicament having selective alpha 2 activity, thus achieving the advantages of using as single selective alpha 2 agonist. See U.S. Patent Application 10/152,424.

This new method is particularly effective when administered as a prophylactic treatment, i.e. before damage to the nerve has taken place, or before long-term progression of the disease state, such as Alzheimer's or Parkinson's disease, has taken place. Without wishing to be held to a particular theory regarding the role that the compounds of the present invention play in neuroprotection, Applicants hypothesize that the compounds and methods described may stimulate the production of certain factors of the bcl-2 family; the increased expression of such factors has been measured by the increased expression of mRNA encoding their production; these factors (bcl-2 and bcl-XL can suppress the apoptotic program. These factors can counterbalance the presence or induction of bcl-2 apoptosis factors such as bad and bax which may be produced as a result of noxious provocations to the nerve cells. Thus, it is further contemplated that the compounds of the present invention which provide cell survival signals to the nerve can advantageously be used in combination with compounds that inhibit cell death. Such cell death inhibiting compounds include NMDA antagonists, especially memantine, which block excitotoxic effects of excess glutamate; nitric oxide synthetase inhibitors; free-radical scavengers and calcium channel blockers.

Any suitable method of administering the presently useful compound or compounds to the mammal to be treated may be used. In all the methods, the preferred mammal is a human. The particular method of administration chosen is preferably one which allows the presently useful compound or compounds to have the desired therapeutic effect in an effective manner, e.g., low effective concentration and low incidence of side effects.

Administration of the presently useful compounds for use in the methods of this invention can include, oral, parenteral, intravenous, subcutaneous and other modes of systemic administration. The compounds are administered in a therapeutically effective amount either alone or in combination with a suitable pharmaceutically acceptable carrier or excipient.

Depending on the intended mode of administration, the presently useful compound or compounds may be incorporated in any pharmaceutically acceptable dosage form, such as for example, tablets, suppositories, pills, capsules, powders, liquids, solutions, infusions, suspensions, emulsions, aerosols or the like, preferably dosage forms suitable for single administration of precise dosages, or sustained release dosage forms for continuous controlled administration. Preferably, the dosage form will include a pharmaceutically acceptable excipient and the presently useful compound or compounds and, in addition, may contain other medicinal agents, pharmaceutical agents, carriers, adjutants, etc.

For solid dosage forms, non-toxic solid carriers include pharmaceutical grades of mannitol, lactose, starch, magnesium stearate, sodium saccharin, the polyalkylene glycols, talcum, cellulose, glucose, sucrose and magnesium carbonate. An example of a solid dosage form for carrying out the invention is a suppository containing propylene glycol as the carrier.

Liquid pharmaceutically administrable dosage forms can, for example, comprise a solution or suspension of one or more of the presently useful compounds and optional pharmaceutical adjutants in a carrier, such as for example, water, saline, aqueous dextrose, glycerol, ethanol and the like, to thereby form a solution or suspension. If desired, the pharmaceutical composition to be administered may also contain minor amounts of nontoxic auxiliary substances such as wetting or emulsifying agents, pH buffering agents and the like. Typical examples of such auxiliary agents are sodium acetate, sorbitan monolaurate, triethanolamine, sodium acetate, triethanolamine oleate, etc. Actual methods of preparing such dosage forms are known, or will be apparent, to those skilled in this art; for example, see Remington's Pharmaceutical Sciences, Mack Publishing Company, Easton, Pa., 16th Edition, 1980, . The composition of the formulation to be administered, in any event, contains a quantity of one or more of the presently useful compounds in an amount effective to provide the desired therapeutic effect.

Parenteral administration is generally characterized by injection, either subcutaneously, intramuscularly or intravenously. Injectables can be prepared in conventional forms, either as liquid solutions or suspensions, solid forms suitable for solution or suspension in liquid prior to injection, or as emulsions or infusions. Suitable excipients are, for example, water, saline, dextrose, glycerol, ethanol and the like. In addition, if desired, the injectable or infusible pharmaceutical compositions to be administered may also contain minor amounts of non-toxic auxiliary substances such as wetting or emulsifying agents, pH buffering agents and the like.

The amount of the presently useful compound or compounds administered is, of course, dependent on the therapeutic effect or effects desired, on the specific mammal being treated, on the severity and nature of the mammal's condition, on the manner of administration, on the potency and pharmacodynamics of the particular compound or compounds employed, and on the judgement of the prescribing physician. Generally, the therapeutically effective dosage of the presently useful compound or compounds is preferably in the range of 0.5 or 1 to 100 mg/kg/day.

### DETAILED DESCRIPTION OF THE INVENTION

In one aspect the present invention is drawn to the use in methods for treating Alzheimer's disease as Parkinson's disease comprising administering to the brain of a mammal in need thereof a therapeutically effective amount of a selective alpha 2 adrenergic receptor agonist.

In connection thereto the Applicants have discovered that selective or specific alpha 2 receptor agonists have an unexpectedly increased efficacy over compounds that have alpha 1 receptor agonist activity. Applicants believe that stimulation of the alpha 1 adrenergic receptor results in interference with the neuroprotective activity of such agents such that any sedative effect that such agent may have has an EC50 similar, or within about a three-fold range of the neuroprotective activity for such compound. Thus, any neuroprotective activity provided by such non-selective agents is seen at concentrations that will tend to sedate or be toxic to the patient. In part for this reason, alpha adrenergic agents have not generally been used as neuroprotective agents in the past, except in topical or local applications (such as ophthalmic applications) in which the agent is not generally administered systemically.

Other compound types having selective or specific alpha 2B or alpha 2B/2C activity are described in co-owned U.S. patent applications 09/794,874 and 10/153,328.

While not wishing to be limited by theory, the present applicants believe that most or the entire neuroprotective efficacy of these agents are provided by stimulation of the alpha 2B and/or alpha 2C receptors. The brain has not generally been thought to be rich in alpha 2B or 2C receptors. However, the applicants have found that the agents and methods of the present invention can provide neuroprotective effects to neurons projecting from or to the substantia nigra and the ventral tegmental area of the brain, and believe that these effects may also be seen in neurons of the locus coruleus, which project into the cortex. Thus, the agents described herein are useful in the treatment of conditions such as Alzheimer's disease and Parkinson's disease.

### Example 1

### Methods:

### Open-Field Test

Each animal is placed in a clear plastic open field box with two rows of photo-beams mounted on the sides to distinguish be5tween horizontal (i.e. distance traveled) and vertical (i.e. "rears") movements. Ambient conditions are low noise and dim lighting. The animal's movements within the box are measured for 5 minutes, and calculated from records of the number and type of "beam-breaks" or photo-beam crossings. On the last Open Field test, only rears are counted, and classified as either "supported" or "unsupported". Supported rears are when an animal places at least one forelimb on the side wall of the box when a rear is recorded. In an unsupported rear the mouse is supported solely by hindlimbs. These rears are distinguished by videorecording. The number of unsupported rears is the most reliable measure of dopamine neuron loss in this test.

### Tail Hanging test

Mice are hung by their tails 3 times each, for approximately 10 seconds each time. Each mouse is hung by the base of its tail about 30 cm above the surface of a table until the mouse turns either left or right. A left turn is given a score of 0 and a right turn is given a score of 1.

Forelimb placement is also noted during tail hanging. The placement of the limbs is given a score on a 4 point scale. Extended limbs, or those placed above the head are given a score of 0. Limbs which are clasped or held against the body are given a score of 3. Scores of 1 or 2 are assigned for relative stages between the two extremes. Hindlimb placement is also scored as below.

### Nest Building

4 mice of the same gender from one group are placed in a plastic tub. Eight strips of paper towel are placed in a neat pile at the front of the tub. The nests build from the paper towel are scored at 24 hours, 48 hours, 72 hours and 96 hours following treatment. Scores are assigned as follows: 0 = paper shredded and formed into a full nest with overhead cover; 1 = paper shredded and formed into a full nest without overhead cover; 2 = paper slightly shredded or chewed and loosely gathered into one area; 3 = paper slightly chewed with no apparent gathering; and 4 = no apparent gathering.

### Neuron Counting

Between 55 and 60 day post MPTP injection, the mice are sacrificed using sodium nembutol. Mouse brains are perfused with phosphate buffered saline followed by Lana's fixative (paraformaldehyde and picric acid). The brains are removed and placed in Lana's fixative for 7 to 10 days. The brains are then sectioned coronally at 50 micrometers using a vibratome. And the sections stained with an antibody to tyrosine hydoxylase, the rate limiting enzyme in dopamine synthesis. The section was then examined under microscope at 100X magnification. Tissue slices (-2.9 mm and -3.6 mm posterior to Bregma were selected for cell counting in the SN and VTA. These sections are at the midpoint of the rostral half and the midpoint of the cauldual half of SN, respectively. Each TH-labeled cell that is clearly visible that has between 2 and 6 neurites is considered a neuron. An overall average count for each animal is calculated from the four sections (rostra1 and caudal; left and right). An average is obtained for the four sections. Separate analyses are performed on the neuron counts from SN and VTA. These counts are analyzed using a repeated measures ANOVA, following by tests of the between groups effect using Fisher's HSD method.

### Experimental Procedure

Mice that receive systemic injections of the pyridine toxin 1-methyl-4-phenyl-1,2,3,6-tetrahydropyridine (MPTP) selectively lose large numbers of dopaminergic neurons in the substantia nigra (SN) and the ventral tegmental area (VTA). Loss of dopamine cells in the SN mimics the clinical condition seen in Parkinson's disease. Loss of such cells in the VTA may contribute to the cognitive deficits seen in Parkinson's and Alzheimer's disease, due to these neurons' projections into the frontal cortex.

50 C57B1/B6 type mice (8-12 weeks old) are allowed to acclimate for 12-14 days before experimental use. The mice are then randomly assigned to the following groups: MPTP plus DMSO vehicle; vehicle alone, MPTP plus brimonidine(3mg/kg/day), MPTP plus AGN 197075(3mg/kg/day), and MPTP plus AGN 196923(3mg/kg/day)).

The structure of 197075 is as follows: 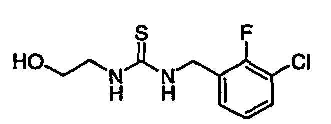 and synthesis information for this compound can be found in US Patent No:6,313,172.

The structure of 196923 is as follows: 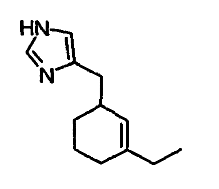 and synthetic information concerning this compound can be found in US patent application serial number 09/815,362, which shares ownership with this patent application.

In the presently described assays, each mouse is then given an initial Open-Field test and a Tail Hanging test. The Open Field test is the most frequently used behavioral assay of MPTP-treated mice, and appears to be sensitive to loss of dopaminergic input from the substantia nigra. The Tail-Hanging test is sensitive to direct striatal damage; the Nest Building test is sensitive to loss of striatal input from the frontal cortex.

Four out of the 5 groups of mice receive an infusion of a test compound (or of vehicle containing no compound). These infusions are administered via subcutaneous implanted osmotic mini-pump over a 14 day period at a flow of 0.25 microliters/hour. Three days following implantation of the pumps, the mice are given the Open Field and Tail Hanging tests, along with the group of control mice, who are not implanted with mini-pumps. Immediately following the tests, the pump containing mice were given a 40 mg/kg injection of MPTP subcutaneously. All groups are then given the Open Field and Tail Hanging tests at 10-12 days and 30-40 days following MPTP treatment. The Open Field, and Tail Hanging tests are given at 50-55 days post MPTP treatment.

For all behavioral tests, the results are first analyzed using a repeated measures ANOVA, following by tests of the between groups effect using Fisher's HSD method.

### Results

### Open Field test

Prior to MPTP treatment there is no significant difference among the 5 groups in distance traveled or number of rears.

The vehicle group is significantly more active than controls at 10 and 30 days post MPTP treatment. The brimonidine treated mice show no alteration in this increase of activity; thus there is no significant difference between this group and the vehicle group. AGN 196923 significant reduces the amount of activity (as compared to the vehicle), and these mice are indistinguishable from the control group. The AGN 197075 group shows a similar trend in the same direction. Neither AGN compound is significantly different from the vehicle at 30 days post MPTP.

MPTP treatment appears to cause a reduction in the total number of rears at 10 days post MPTP. At 30 days there is no MPTP effect on total rearing (as compared with the vehicle group), and only a slight reduction in rearing versus the control group.

At 50-60 days post MPTP treatment, mice that receive AGN 197075 make significantly more unsupported rears than mice that receive vehicle. The vehicle group make fewer unsupported rears than normal mice. There is no effect of either MPTP or the compounds on supported rearing.

### Tail Hang test

No significant group differences are observed in the tail hang test prior to MPTP treatment, and none are seen post MPTP in the hindlimbs. MPTPO significantly impaired forelimb extensions at all three post-lesion time points. Thus, this impairment is not reversed over time. AGN 197075 significantly reduces this impairment during the period that the compound is administered. AGN 196923 and brimonidine show no such trend. However, all three compounds tend to reduce the impairment when measured post-dosing (i.e. after 14 days of compound administration).

### Neuron Count

In the substantia nigra, MPTP-treated animals have 58% fewer neurons than untreated animals. However those that receive brimonidine, AGN 196923, or AGN 197075 have significantly less neuron loss.

| **Treatment Group** | **Average Neuron Count (statistical error)** |
|---|---|
| *Control* | 60 (± 5) |
| *Vehicle + MPTP* | 25 (± 2) |
| *AGN 196923 + MPTP* | 35 (± 2) |
| *AGN 197075 + MPTP* | 37 (± 2) |
| *Brimonidine* | 32 (± 1) |

In the Ventral Tegmental area, MPTP was found to result in an average decrease of 28% fewer neurons than in the Control group. Each of the test compounds (AGN 197075, AGN 196923 and brimonidine) reduced this loss by an average of about 10%.

## Claims

1. Use of a selective alpha 2B or selective alpha 2B/2C adrenergic receptor agonist in the manufacture of a medicament for the treatment of a neurodegenerative condition of the brain causing damage to neurons projecting to or from the substantia nigra, wherein said neurodegenerative condition is Parkinson's or Alzheimer's disease.

2. The use of claim 1 wherein said medicament containing said selective alpha 2B or selective alpha 2B/2C adrenergic receptor agonist is administered to the brain of said mammal by systemic delivery.

3. The use of claim 2 wherein administration of said medicament is effective two prevent death or degeneration of neurons projecting to or from the ventral tegmental area.

4. The use of any one of claims 2 - 3 wherein said compound is an optionally substituted compound selected from the group consisting of an imidazoline, a thiourea, a thione, a quinoxaline and an imidazolone.

5. The use of claim 1 in which said selective alpha 2B or selective 2B/2C adrenergic receptor agonist compound is a specific alpha 2B or specific alpha 2B/2C adrenergic receptor agonist.

6. The use of claim 5 in which said compound is an optionally substituted compound selected from the group consisting of an imidazoline, a thiourea, a thione, a quinoxaline and an imidazolone.

7. The use of claim 1 wherein the amount of sedation accompanying administration of said drug at a given degree of therapeutic efficacy is less than that accompanying administration of a dose of dexmedetomidine at the same degree of therapeutic efficacy.

8. Use of a selective alpha 2B or 2B/2C adrenergic receptor agonist in the manufacture of a medicament for treatment of a condition involving neurodegeneration of brain tissue, wherein said condition, is Parkinson's or Alzheimer's disease.

9. The use of claim 8, wherein the selective alpha 2 adrenergic receptor agonist is at least about 12 times more selective for an 2B or 2B/2C adrenergic receptor, in comparison to an alpha 1 receptor, than dexmedatomidine.

10. The use of claim 9 wherein said selective alpha 2 receptor agonist is a compound selected from the group of compounds consisting of an imidazoline, a thiourea, a thione, a quinoxaline and an imidazolone.

11. The use of any of claims 8-10 wherein said selective alpha 2B or alpha 2B/2C receptor agonist is a specific alpha 2 receptor agonist.

12. The use of any of claims 8-10 wherein said selective alpha 2B or alpha 2B/2C receptor agonist is a specific alpha 2B or alpha 2B/2C receptor agonist.

13. The use of claim 12 wherein said specific alpha 2B or alpha 2B/2C receptor agonist is a specific alpha 2 receptor agonist.

## Patentansprüche

1. Verwendung eines selektiven alpha 2B oder selektiven alpha 2B/2C-adrenergen Rezeptoragonisten bei der Herstellung eines Medikaments zur Behandlung eines neurodegenerativen Zustands des Gehirns, der eine Schädigung von Neuronen verursacht, die aus dem Substantia nigra hervorstehen oder hierauf hinweisen, wobei der neurodegenerative Zustand die Parkinson- oder die Alzheimer-Krankheit ist.

2. Verwendung gemäss Anspruch 1, worin das Medikament, das den selektiven alpha 2B oder selektiven alpha 2B/2C-adrenergen Rezeptoragonisten enthält, dem Gehirn des Säugers durch systemische Zulieferung verabreicht wird.

3. Verwendung gemäss Anspruch 2, worin die Verabreichung des Medikaments wirksam ist, um den Tod oder die Degenerierung von Neuronen zu vermeiden, die aus dem ventralen tegmentalen Bereich hervorstehen oder hierauf hinweisen.

4. Verwendung gemäss irgendeinem der Ansprüche 2 bis 3, worin die Verbindung eine gegebenenfalls substituierte Verbindung ist, ausgewählt aus der Gruppe bestehend aus einem Imidazolin, einem Thioharnstoff, einem Thion, einem Chinoxalin und einem Imidazolon.

5. Verwendung gemäss Anspruch 1, worin die selektive alpha 2B oder selektive alpha 2B/2C-adrenerge Rezeptoragonistverbindung ein spezifischer alpha 2B oder ein spezifischer alpha 2B/2C-adrenerger Rezeptoragonist ist.

6. Verwendung gemäss Anspruch 5, worin die Verbindung eine gegebenenfalls substituierte Verbindung ist, die ausgewählt ist aus der Gruppe bestehend aus einem Imidazolin, einem Thioharnstoff, einem Thion, einem Chinoxalin und einem Imidazolon.

7. Verwendung gemäss Anspruch 1, worin die Menge der Beruhigung, die mit der Verabreichung der Arznei bei einem gegebenen Grad therapeutischer Wirksamkeit einhergeht, kleiner ist als die, die mit der Verabreichung einer Dosis von Dexmedetomidin beim gleichen Grad der therapeutischen Wirksamkeit einhergeht.

8. Verwendung eines selektiven alpha 2B oder 2B/2C-adrenergen Rezeptoragonisten bei der Herstellung eines Medikaments zur Behandlung eines Zustands, der die Neurodegenerierung von Gehirngewebe involviert, worin der Zustand die Parkinson- oder die Alzheimer-Krankheit ist.

9. Verwendung gemäss Anspruch 8, worin der selektive alpha 2-adrenerge Rezeptoragonist mindestens etwa 12 mal selektiver für einen 2B oder 2B/2C-adrenergen Rezeptor, im Vergleich zu einem alpha 1-Rezeptor, als Dexmedatomidin ist.

10. Verwendung gemäss Anspruch 9, worin der selektive alpha 2-Rezeptoragonist eine Verbindung ist, die ausgewählt ist aus der Gruppe von Verbindungen bestehend aus einem Imidazolin, einem Thioharnstoff, einem Thion, einem Chinoxalin und einem Imidazolon.

11. Verwendung gemäss irgendeinem der Ansprüche 8 bis 10, worin der selektive alpha 2B oder alpha 2B/2C-Rezeptoragonist ein spezifischer alpha 2-Rezeptoragonist ist.

12. Verwendung gemäss irgendeinem der Ansprüche 8 bis 10, worin der selektive alpha 2B oder alpha 2B/2C-Rezeptoragonist ein spezifischer alpha 2B oder alpha 2B/2C-Rezeptoragonist ist.

13. Verwendung gemäss Anspruch 12, worin der spezifische alpha 2B oder alpha 2B/2C-Rezeptoragonist ein spezifischer alpha 2-Rezeptoragonist ist.

## Revendications

1. Utilisation d'un agoniste sélectif du récepteur adrénergique alpha 2B ou sélectif du récepteur alpha 2B/2C pour la préparation d'un médicament destiné à traiter une affection neurodégénérative du cerveau entraînant des lésions des neurones se projetant dans ou à partir du locus niger, l'affection neurodégénérative étant la maladie de Parkinson ou la maladie d'Alzheimer.

2. Utilisation selon la revendication 1, dans laquelle ledit médicament contenant ledit agoniste sélectif du récepteur adrénergique alpha 2B ou sélectif du récepteur alpha 2B/2C est administré au cerveau dudit mammifère par voie systémique.

3. Utilisation selon la revendication 2, dans laquelle l'administration dudit médicament est efficace pour prévenir la mort ou la dégénérescence des neurones se projetant dans ou à partir de l'aire tegmentale ventrale.

4. Utilisation selon l'une des revendications 2 à 3, dans laquelle ledit composé est un composé éventuellement substitué choisi dans le groupe constitué d'une imidazoline, une thio-urée, une thione, une quinoxaline et une imidazolone.

5. Utilisation selon la revendication 1, dans laquelle ledit composé agoniste sélectif du récepteur adrénergique alpha 2B ou sélectif du récepteur alpha 2B/2C est un agoniste spécifique du récepteur adrénergique alpha 2B ou alpha 2B/2C.

6. Utilisation selon la revendication 5, dans laquelle ledit composé est un composé éventuellement substitué choisi dans le groupe constitué d'une imidazoline, une thio-urée, une thione, une quinoxaline et une imidazolone.

7. Utilisation selon la revendication 1, dans laquelle la quantité de sédation accompagnant l'administration dudit médicament à un degré donné d'efficacité thérapeutique est inférieure à celle accompagnant l'administration d'une dose de dexmédétomidine au même degré d'efficacité thérapeutique.

8. Utilisation d'un agoniste sélectif du récepteur adrénergique alpha 2B ou 2B/2C pour la préparation d'un médicament destiné à traiter une affection comprenant la neurodégénérescence des tissus cérébraux, ladite affection étant la maladie de Parkinson ou la maladie d'Alzheimer.

9. Utilisation selon la revendication 8, dans laquelle l'agoniste sélectif du récepteur adrénergique alpha 2 est au moins environ 12 fois plus sélectif qu'un récepteur adrénergique 2B ou 2B/2C comparé à un récepteur alpha 1 par rapport à la dexmédatomidine.

10. Utilisation selon la revendication 9, dans laquelle ledit agoniste sélectif du récepteur alpha 2 est un composé choisi dans le groupe de composés constitué d'une imidazoline, une thio-urée, une thione, une quinoxaline et une imidazolone.

11. Utilisation selon l'une quelconque des revendications 8 à 10, dans laquelle ledit agoniste sélectif du récepteur alpha 2B ou alpha 2B/2C est un agoniste spécifique du récepteur alpha 2.

12. Utilisation selon l'une des revendications 8 à 10, dans laquelle ledit agoniste sélectif du récepteur alpha 2B ou alpha 2B/2C sélectif est un agoniste spécifique du récepteur alpha 2B ou alpha 2B/2C.

13. Utilisation selon la revendication 12, dans laquelle ledit agoniste spécifique du récepteur alpha 2B ou alpha 2B/2C est un agoniste spécifique du récepteur alpha 2.
